(19) Europäisches Patentamt
European Patent Office
Office européen des brevets

(11) Publication number: **0 320 006 A2**

(12) # EUROPEAN PATENT APPLICATION

(21) Application number: **88120640.3**

(51) Int. Cl.4: **A61K 31/44**

(22) Date of filing: **09.12.88**

The title of the invention has been amended (Guidelines for Examination in the EPO, A-III, 7.3).

(30) Priority: **11.12.87 US 131778**

(43) Date of publication of application: **14.06.89 Bulletin 89/24**

(84) Designated Contracting States: **AT BE CH DE ES FR GB GR IT LI LU NL SE**

(71) Applicant: **LABORATOIRES SYNTEX**
**20 rue Jean Jaurès**
**F-92807 Puteaux, Hauts de Seine(FR)**

(72) Inventor: **Selkirk, Alastair B.**
**14 Cramond Glebe Gardens**
**Edinburgh Scotland(GB)**
Inventor: **Alps, Brian J.**
**2 St. Ninians Avenue**
**Linlithgow Scotland(GB)**
Inventor: **Armstrong, John M.**
**14, Allée des Eaux Farouches**
**F-9190 Gif Sur Yvette(FR)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**D-8000 München 40(DE)**

(54) **Uses of 1,4-dihydropyridines.**

(57) Compounds of Formula 1 are useful for treating convulsive disorders, irritable bowel syndrome, and for protecting neural tissue from ischemic damage:

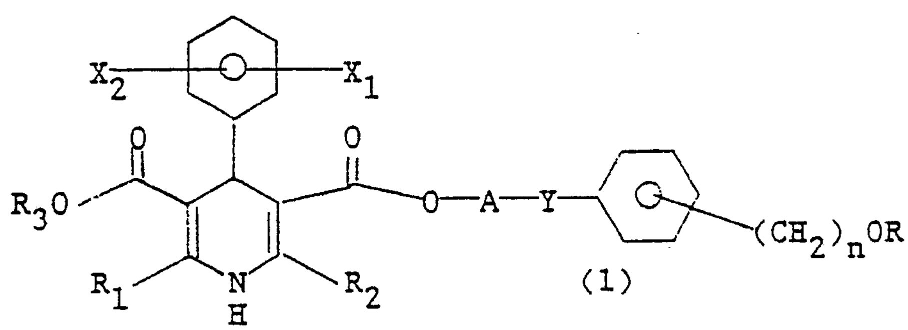

(1)

wherein
n is an integer from 1 to 4;
$R_1$ and $R_2$ are lower alkyl;
$R_3$ is lower alkyl or alkoxyalkyl;
A is alkylene of two to eight carbon atoms;
$X_1$ and $X_2$ are each independently $-NO_2$, $-CF_3$, $CH_3O-$, -CN, -H, lower alkyl or halo;

EP 0 320 006 A2

Y is -O-, -S-, -S(O)-, or $-S(O)_2-$; and
R is H, lower alkyl, cycloalkyl, alkoxyalkyl, cycloalkyloxy-alkyl, alkoxycycloalkyl, acyl, or saturated or unsaturated 5- or 6-membered heterocyclyl optionally substituted with lower alkyl or alkoxy, wherein the heteroatom is one oxygen atom.

# NEUROPROTECTIVE COMPOUNDS

This invention relates to the use of certain 1,4-dihydropyridine derivatives to protect neural tissue against ischemic damage, to treat convulsive disorders and to treat irritable bowel syndrome.

## Related Disclosures

Ischemic damage to neurons occurs when oxygenation is interrupted for periods longer than several minutes (infarction). Such interruptions may occur during a cerebrovascular accident, or "stroke." Stroke is the most common cause of neurological disability in Western countries. Stroke is frequently caused by thrombosis or embolisim in the brain, interrupting the flow of blood.

Convulsive disorders, including epilepsy, are generally caused by an acute focal or generalized disturbance in cerebral function. Common causes include hyperpyrexia (from acute infection or heat stroke), direct CNS infection (e.g., meningitis), metabolic disturbance (e.g., hypoglycemia, hypoparathyroidism, phenylketonuria), toxic agents (e.g., camphor, lead, pentylenetetrazol, strychnine), cerebral hypoxia (e.g., carbon monoxide poisoning), brain lesions (e.g., intracrania hemorrhage, neoplasm), congenital brain defects, cerebral trauma (e.g., skull fracture), anaphylaxis (e.g., drug allergy), and cerebral infarct or hemorrhage. Idiopathic epilepsy, which accounts for 75% of all epilepsy patients, has no discernable cause. Epilepsy affects about 2% of the population, with about 90% of those patients affected with grand mal seizures.

Irritable bowel syndrome, of which one form is "spastic colon," is a disorder of the lower intestine. The syndrome is often associated with colonic pain, constipation, and/or diarrhea. It may lead to diverticular disease.

Certain 4-aryl-1,4-dihydropyridine derivatives are known calcium entry antagonists. See, for example, U.S. Patent Nos. 3,485,847 and 4,044,141. The compounds of formula 1 are also useful for the treatment of cardiovascular diseases such as hypertension, congestive heart failure, angina, migraine, and vasospastic disorders. Their preparation and administration for treatment of cardiovascular disorders is described in U.S. Patent Application Serial No. 874,264, filed June 13, 1986, issued August 2, 1988 as U.S. Pat. No. 4,761,420, incorporated herein in full by reference, which has a European equivalent (application 87108580.9 which published December 16, 1987 as publication no. 249,245). This application includes administration of the compounds orally, systemically (e.g., transdermally, intranasally or by suppository) or parenterally (e.g., intramuscularly, subcutaneously and intravenously).

It has now been found that 4-aryl-1,4-dihydropyridine derivatives of Formula 1 are useful for protecting neurons from ischemic damage, for preventing convulsions, and for treating irritable bowel syndrome.

One aspect of the invention is a method for protecting neural tissue against ischemic damage, which method comprises administering to a subject susceptible to stroke an effective amount of a compound of formula 1:

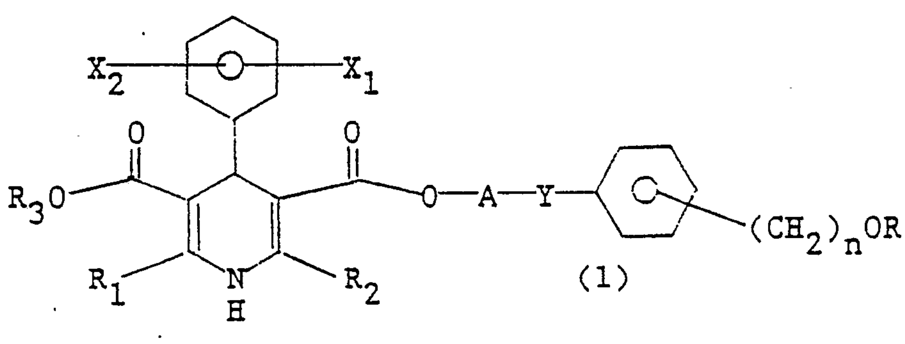

(1)

wherein
n is an integer from 1 to 4;
$R_1$ and $R_2$ are lower alkyl;
$R_3$ is lower alkyl or alkoxyalkyl;
A is alkylene of two to eight carbon atoms;
$X_1$ and $X_2$ are each independently $-NO_2$, $-CF_3$, $CH_3O-$, -CN, -H, lower alkyl or halo;

Y is -O-, -S-, S(O)-, or $S(O)_2$-; and

R is H, lower alkyl, cycloalkyl, alkoxyalkyl, cycloalkyloxy-alkyl, alkoxycycloalkyl, acyl, or saturated or unsaturated 5- or 6-membered heterocyclyl optionally substituted with lower alkyl or alkoxy, wherein the heteroatom is one oxygen atom.

Another aspect of the invention is a method for treating convulsive disorders, which method comprises administering to a subject susceptible to convulsive disorders an effective amount of a compound of formula 1.

Another aspect of the invention is a method for treating irritable bowel syndrome, which method comprises administering to a subject susceptible to irritable bowel syndrome an effective amount of a compound of formula 1.

Another aspect of the invention is use of a compound of formula 1 to manufacture a medicament.

Another aspect of the invention is as an agent for the treatment of irritable bowel syndrome, convulsive disorders, or the protection of neural tissue against ischemic damage.

Another aspect of the invention is a composition for treating irritable bowel syndrome, convulsive disorders, or the protection of neural tissue against ischemic damage.

Another aspect of the invention is a process for the production of a medicament based on a compound of formula 1 against irritable bowel syndrome, convulsive disorders, or for protection of neural tissue from ischemic damage.

Another aspect of the invention is a process for preparing a composition for treating irritable bowel syndrome, convulsive disorders, or protection of neural tissue from ischemic damage.

## DEFINITIONS

As used in the specification and appended claims, unless specified to the contrary, the following terms have the meaning indicated:

The term "lower alkyl" refers to a straight or branched chain monovalent radical consisting solely of carbon and hydrogen, containing no unsaturation and having from one to four carbon atoms. Examples of lower alkyl groups are methyl, ethyl, n-propyl, i-propyl, n-butyl, i-butyl, s-butyl and t-butyl.

The term "alkylene" refers to a straight chain divalent radical of the form $-(CH_2)_x-$, where x is an integer from 2 to 8. Examples of alkylene radicals include ethylene, propylene, butylene, pentalene, hexylene, heptalene, and octalene.

The term "cycloalkyl" refers to a cyclic hydrocarbon radical containing no unsaturation and having from three to eight carbon atoms. Examples of cycloalkyl groups are cyclopropyl, cyclohexyl, cyclohexylmethyl, 3-methylcyclopentyl and the like.

The term "alkoxy" refers to a radical of the form $R_aO$-, where $R_a$ is lower alkyl as defined above.

The term "alkoxyalkyl" refers to radicals of the form $-R_b-O-R_a$, where $R_b$ is alkylene of one to six carbon atoms, and $R_a$ is lower alkyl as defined above. Examples of alkoxyalkyl groups are methoxymethyl, methoxyethyl, 2-(2-propoxy)ethyl, t-butoxymethyl, and the like.

The term "cycloalkyloxy alkyl" refers to radicals of the form $-R_b-O-R_c$, where $R_c$ is cycloalkyl and $R_b$ is alkylene of one to six carbon atoms, as defined above. Examples of cycloalkyloxy-alkyl groups are cyclohexyloxy-methyl, cyclohexyloxy-ethyl, cyclopentyloxy-methyl, cyclohexyloxy-butyl, cyclopropyloxy-methyl, and cyclooctyloxy-butyl.

The term "alkoxy-cycloalkyl" refers to radicals of the form $-R_c-O-R_a$, where $R_c$ is cycloalkylene (a divalent cycloalkyl radical of three to eight carbon atoms) and $R_a$ is lower alkyl, as defined above. Examples of alkoxy-cycloalkyl groups are 4-methoxy-cyclohexyl, 3-ethoxy-cyclopentyl, methoxy-cyclopropyl, and 5-butoxy-cyclooctyl.

The term "halo" as used herein refers to fluoro, chloro, bromo and iodo.

The term "heterocyclyl" as used herein refers to saturated and unsaturated cyclic groups composed of carbon, hydrogen, and one oxygen heteroatom. Heterocyclyl groups as referred to herein are rings of 5 or 6 members. Examples of saturated heterocyclyl radicals are tetrahydrofuranyl and tetrahydropyranyl. Examples of unsaturated heterocyclyl groups are furanyl, dihydrofuranyl, pyranyl, and the like. Preferred heterocyclyl groups are tetrahydropyran-2-yl, and tetrahydrofuran-2-yl, especially tetrahydropyran-2-yl.

The term "optionally substituted" includes the cases where a group is substituted or unsubstituted. As used herein, "optionally substituted" heterocyclyl groups may be substituted with zero, one or two lower alkyl or alkoxy radicals, as those terms are defined herein. Thus, the term "optionally substituted heterocyclyl radical" includes, for example, 2-methyltetrahydrofuran-4-yl, 2-methoxytetrahydropyran-4-yl, 2-methylfuran-4-yl, and the like.

The term "acyl" as used herein refers to groups of the formula $R_aC(O)$-, where $R_a$ is lower alkyl as defined above.

The term "treatment" as used herein covers any treatment of a disease in a mammal, particularly a human, and includes:

(i) preventing the disease from occurring in a subject which may be predisposed to the disease but has not yet been diagnosed as having it;

(ii) inhibiting the disease, i.e., arresting its development; and

(iii) relieving the disease, i.e., causing regression of the disease. For example, administration of a compound of formula 1 to reduce or eliminate convulsions in a patient suspected of suffering from epilepsy falls within the definition of treatment herein. Similarly, administration of a compound of formula 1 to minimize neurological damage in a patient at risk of suffering a stroke falls within the definition of treatment.

The nomenclature used herein is a modified form of the I.U.P.A.C. convention. Compounds of the invention are named as derivatives of 1,4-dihydropyridine. The positions in the compounds are numbered beginning with the pyridine nitrogen and proceeding clockwise in all drawings of the structure. For example, the following compound is named 2,6-dimethyl-3-carbomethoxy-4-(3-nitrophenyl)-5-(3-[4-(2-methoxyethyl)-phenoxy]propoxycarbonyl)-1,4-dihydropyridine:

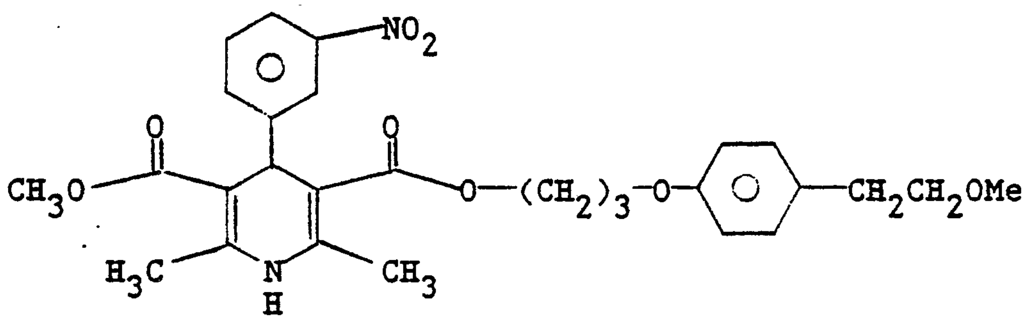

Compounds of formula 1 have a chiral center at C4 in the dihydropyridine ring, and thus can exist as optical isomers. Certain compounds in which R is asymmetric will thus exist as mixtures of diastereomers. In the compounds of the invention, any isomer or mixture of isomers may be used. The isomers may be separated by various methods, for example selective crystallization and column chromatography. See for example T. Shibanuma, et al., Chem. Pharm. Bull., 28, 2809-2812 (1980). Alternatively, the compounds of the invention may be prepared using optically active reactants, or by a combination of separation and chiral synthesis. The invention includes all optical isomers of any asymmetric compound of formula 1, as well as mixtures thereof. Optical isomers of compounds may be specified (+) or (-), indicating the direction the chiral center rotates a plane of polarized light. Compounds of the invention which have multiple chiral centers are prepared by condensation of one or more optically active intermediates and are specified by indicating the sign of rotation of the optically active intermediates and which dihydropyridine isomer from which the compound is prepared.

Optically active intermediates and compounds of formula 1 may also be designated using the IUPAC R-S convention, sometimes called the "sequence rule." A description of the R-S convention may be found, for example, in "Introduction to Organic Chemistry" by A. Streitwieser, Jr. and C. Heathcock, (Macmillan Pub. Co., New York, 1976), pages 110-114. Where a compound has more than one chiral center, the C4 (dihydropyridine ring) center is indicated first, with the remaining chiral centers indicated in order of increasing distance from the dihydropyridine ring. Preferred compounds of the invention have the (S) configuration at C4. An indication such as (S,RS) refers to a mixture of isomers having the (S) configuration at C4 and a mixture of (R) and (S) isomers at the tetrahydropyranyl radical chiral center (*).
For example, the compound depicted below is (S,RS)-2,6-dimethyl-3-carbomethoxy-4-(3-nitrophenyl)-5-[3-(4-[2-(tetrahydropyran-2-yloxy)ethyl]phenoxy)propoxy carbonyl]-1,4-dihydropyridine:

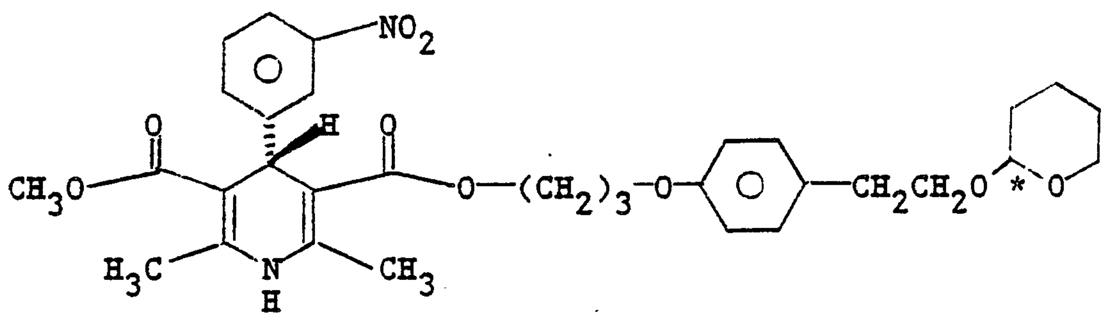

## ADMINISTRATION AND FORMULATION

The use of the compounds of formula 1 to protect against ischemic damage is experimentally demonstrated by inducing ischemic damage in a suitable test animal (e.g., gerbil), followed by examination of the brain (particularly selectively vulnerable $CA_1$ hippocampal cells) for living and dead cells. This experimental test is described in the literature by Kirino, T. Brain Research, 239, 57-69 (1982). A detailed description is set forth in Example 3.

Anticonvulsive activity of the compounds of Formula 1 is demonstrated by administering the test compound to an experimental animal, then administering a convulsant drug such as pentylenetetrazol. The amount of test compound needed to prevent convulsions induced by a fixed amount of pentylenetetrazol can then be determined. This experimental test is described in the literature by Krall, R.J., et al, Epilepsia, 19, 409-428 (1978). A detailed description is set forth in Example 2, hereinafter.

The use of compounds of Formula 1 for treating irritable bowel syndrome is assessed by a method modified from Macht and Barba-Gose, J. Amer. Pharm. Assn., 20, 559 (1931), by measuring the transit time of charcoal meal through mouse intestine stimulated with barium chloride. A detailed description is set forth in Example 4, hereinafter.

Compositions containing a therapeutically effective amount of a compound of formula 1 are useful for treating convulsive disorders (such as epilepsy), irritable bowel syndrome, and for protecting neural tissue against ischemic damage. Such compositions comprise a therapeutically effective amount of a compound of formula 1, in admixture with a pharmaceutically acceptable non-toxic carrier. A therapeutically effective amount is that amount which, when administered to a mammal in need thereof, is sufficient to effect treatment, as defined above. Thus, the level of the drug in the formulation can vary from about 5 percent weight (%w) to about 95%w of the drug based on the total formulation and about 5%w to 95%w excipient. Preferably the drug is present at a level of about 10%w to about 70%w.

Useful pharmaceutical carriers for the preparation of the pharmaceutical compositions hereof can be solids or liquids. Thus, the compositions can take the form of tablets, pills, capsules, powders, sustained release formulations, solutions, suspensions, elixirs, aerosols, and the like. Carriers can be selected from the various oils, including those of petroleum, animal, vegetable or synthetic origin, for example, peanut oil, soybean oil, mineral oil, sesame oil, and the like. Water, saline, aqueous dextrose solutions, and glycols are preferred liquid carriers, particularly for injectable solutions. Suitable pharmaceutical excipients include starches, celluloses, talc, glucose, lactose, sucrose, gelatin, magnesium stearate, glycerol monostearate, sodium chloride, polyethylene glycol, glycerol, propylene glycol, water, ethanol, and the like. Other suitable pharmaceutical carriers and their formulations are described in "Remington's Pharmaceutical Sciences" edited by A. Osol, l6th edition, Mack Publishing Company, 1980.

In the practice of the method of the present invention, a therapeutically effective amount of the compound of formula 1 or a pharmaceutical composition containing same is administered via any of the usual and acceptable methods known in the art, either singly or in combination with another compound or compounds of the present invention or other pharmaceutical agents. These compounds or compositions can thus be administered by injection (e.g. intramuscularly, subcutaneously and intravenously) or by another route, (e.g. transdermally, intranasally, rectally or sublingually). The compounds can be administered either in the form of solid or liquid dosages including tablets, solutions, suspensions, aerosols, and the like, as discussed in more detail above.

The formulation can be administered in a single unit dosage form for continuous treatment or in a single

unit dosage form ad libitum when relief of symptoms is specifically required. Treatment of convulsive disorders and neuroprotection from ischemic damage is best achieved by prophylactic administration, i.e., administration of a "maintenance dose" 1 to 4 times daily. Treatment of irritable bowel syndrome may be effected by prophylactic administration, or by administration ad libitum.

The preferred routes to treat irritable bowel syndrome are oral and rectal. For protecting against ischemic damage, the preferred method is injection, e.g. by intravenous (I.V.) perfusion for emergency situations, then oral administration for continued treatment. For treating convulsions, the preferred treatment for emergencies is I.V. or intramuscular injection, followed by oral administration for continued treatment.

In view of the foregoing as well as in consideration of the degree of severity of the condition being treated, age of subject and so forth, all of which factors are determinable by routine experimentation by one skilled in the art, the effective dosage in accordance herewith can vary over a wide range. Generally, for prophylactic anticonvulsant, or neuroprotective use, a therapeutically effective amount ranges from about 0.001 to about 700 mg per day, preferably from about 0.1 to about 400 mg per day, more preferably from about 1.0 to about 200 mg per day, for an average 70 Kg human. Doses for treating irritable bowel syndrome are similar.

Presently the preferred compound for the treatment of convulsive disorders, the protection against ischemic damage and the treatment of irritable bowel syndrome is the compound of formula 1 wherein n is 2; $R_1$, $R_2$ and $R_3$ are all methyl; A is 1,3-propylene; $X_1$ is 3-nitro; $X_2$ is H; y is O; and R is tetrahydropyranyl, namely [(S,RS)-2,6-dimethyl-3-carbomethoxy-4-(3-nitrophenyl)-5-[3-(4-[2-(tetrahydropyran-2-yloxy)ethyl]phenoxy)propoxycarbonyl]-1,4 dihydropyridine].

## PREPARATION

Compounds of formula 1 are prepared as described in U.S. Patent Application Serial No. 874,264, filed June 13, 1986, issued August 2, 1988 as U.S. Pat. No. 4,761,420, incorporated herein in full by reference, which has a European equivalent (application 87108580.9 which published December 16, 1987 as publication no. 249,245). In general, compounds of formula 1 are prepared by one of ordinary skill in the art using the Hantzch Dihydropyridine Synthesis, once given the instant disclosure and that of S.N. 874,264.

Suitable pharmaceutical formulations may be prepared by standard means by those of ordinary skill in the art.

## EXAMPLE 1

(Formulations)

The following example illustrates the preparation of representative pharmaceutical formulations containing an active compound of formula 1, e.g., (S,RS)-2,6-dimethyl-3-carbo-methoxy-4-(3-nitrophenyl)-5-(3-[4-(2-tetrahydropyran-2-yloxyethyl)phenoxy]propoxycarbonyl)-1,4-dihydropyridine (1).

| A. I.V. FORMULATION | |
|---|---|
| Active compound | 0.01 g |
| Propylene glycol | 20.0 g |
| Polyethylene glycol 400 | 20.0 g |
| Tween 80 | 1.0 g |
| 0.9% Saline solution | qs 100.0 mL |

The active compound is dissolved in propylene glycol, polyethylene glycol 400 and Tween 80. A sufficient quantity of 0.9% saline solution is then added with stirring to provide 100 mL of the I.V. solution which is filtered through a 0.2 micron membrane filter and packaged under sterile conditions.

| B. ORAL DOSE FORMULATIONS | | |
|---|---|---|
| Ingredient | parts by weight | |
| Active compound | 3.33 | 15.00 |
| PEG 600 | 94.25 | 82.875 |
| PEG 4000 | 2.42 | 2.125 |

The active compound is dissolved in the mixture of the glycols heated to about 50° C. This solution is filled into hard gelatin capsules and allowed to cool to form a solid matrix at temperatures of 30 to 35° C.

## EXAMPLE 2

(Anticonvulsant Activity)

(A) Male CD1 mice (Charles River) are randomly divided into groups of at least 20, and treated with 500 µg/Kg of (S,RS)-2,6-dimethyl-3-carbomethoxy-4-(3-nitrophenyl)-5-(3-[4-(2-tetrahydropyran-2-yloxyethyl)-phenoxy]propoxycarbonyl)-1,4-dihydropyridine (the compound is dissolved in 0.1 mL of ethanol and diluted with 99.9 mL saline and injected intraperitoneally) before subcutaneous administration of 100 mg/Kg pentylenetetrazole ("PTZ", a known convulsant) under the following dosing schedules: (A) 15 minutes prior to PTZ administration; (B) 60 minutes prior to PTZ administration; and (C) b.i.d. for three days prior to PTZ administration, a further dose being given on a fourth day 15 minutes prior to PTZ administration. After PTZ administration, the mice are observed for at least 30 minutes, and the incidence of clonic seizures, tonic seizures, and death is noted. The experiment is terminated by pentobarbitone overdose.

The compound demonstrates anticonvulsant activity in this experiment as shown in the following table.

| Compound | Dose Schedule | Number Animals | % Clonic | % Tonic | % Death |
|---|---|---|---|---|---|
| Saline | -- | 40 | 92.5 | 80 | 70 |
| X | A | 21 | 90.5 | 90.5 | 38.1* |
| X | B | 20 | 80 | 40*** | 35* |
| X | C | 20 | 65** | 55 | 60 |

X is (S,RS)-2,6-dimethyl-3-carbomethoxy-4-(3-nitrophenyl)-5-[3-(4-[-(tetrahydropyran-2-yloxy)ethyl]phenoxy)propoxycarbonyl]-1,4-dihydropyridine.

*P is 0.05
**P is 0.02
*** P is 0.01 all vs controls, Chi squared analysis of association.

(B) Similarly, proceeding as in part A above, but substituting other compounds of formula 1, similar anticonvulsant activity is demonstrated.

## EXAMPLE 3

(Neuroprotection from Ischemic Damage)

(A) Reproducible morphological changes characterizing delayed neuronal death ("DND") of selectively

vulnerable $CA_1$ pyramidal cells in the hippocampus following bilateral occlusion of the common carotid arteries can be used to demonstrate neuroprotective effects of a compound against ischemic damage.

Mongolian gerbils of either sex (50-80 g) are treated with a test compound (or control) either intraperitoneally (IP) at 500 mg/kg 15 minutes prior to induction of ischemia followed by B.I.D. dosing for three days post-ischemia, or orally at 5 mg/kg twice daily for 3 out of 4 days and then 15 minutes prior to cerebral ischemia on the 4th day followed by B.I.D. dosing for three days post-ischemia. To facilitate induction of forebrain ischemia the animals are then anesthetized with halothane (5% in 30:70 oxygen:nitrous oxide) delivered by face mask. Following rapid induction, the halothane is decreased to 1.5-2% and maintained at that level for the remainder of the experiment. The left and right common carotid arteries are exposed in the paratracheal region through a ventral midline cervical skin incision and freed from the accompanying vagosympathetic nerve trunks. A loose ligature is placed around the arteries to facilitate identification at the time of occlusion.

The carotid arteries are then bilaterally occluded for 5 minutes using microvascular clips (Ackland C-2-V, Weiss, London). Blood flow is then restored, the wound dusted with antibiotic powder (Cicatrin, Calmic Medical Division, The Wellcome Foundation Ltd., London), and sutured. The subjects are then caged individually and allowed food and water ad libitum.

Seventy-two hours after induction of ischemia, the gerbils are anesthetized with pentobarbitone sodium (4 mg i.p.) and their brains perfuse-fixed by intracardiac injection of 10% buffered formal saline solution. The heads are stored overnight at 4°C in fresh formalin. The brains are removed from the calvarium and fixed for 1 week, prior to being embedded in paraffin wax, sectioned at 7 µm, and stained with cresyl fast violet and haematoxylin-eosin. Brains from normal animals subjected to the same fixation procedure are studied for comparison.

The extent of neuronal death is determined by counting microscopically (at 40x power) all normal and abnormal cells in five fields per hemisphere of the hippocampal $CA_1$ subfield from the paramedian area to its junction with $CA_2$ neurons. The field counts are then summed and the mean percentage of dead cells calculated (n=10 fields per brain). Statistical significance was calculated using the Student's t test for unpaired samples, with significance taken at p less than 0.05.

Compounds of formula 1 show significant neuroprotective activity under this protocol as illustrated in the following table.

| A. intraperitoneal administration | | | |
|---|---|---|---|
| Group | No. of Animals | Percentage abnormal neurone count | No. observations per group |
| normal | 10 | 4.5 ± 0.4 | 100 |
| shams | 7 | 4.6 ± 0.3 | 70 |
| Saline treated controls | 24 | 71.2 ± 2.4 | 240 |
| Compound + alcoholic vehicle | 10 | 5.6 ± 0.3 | 100 |
| alcoholic vehicle alone | 9 | 51.1 ± 4.4 | 90 |
| compound + PEG 300 + water | 9 | 6.6 ± 0.8 | 90 |
| PEG 300 + water vehicle alone | 12 | 62.6 ± 4.0 | 90 |
| B. oral | | | |
| compound + PEG 300 + vehicle | 9 | 58.0 ± 5.0 | 90 |

## EXAMPLE 4

(Irritable Bowel Syndrome Activity)

Conscious mice (15-20g) are treated with vehicle or test compound 15 minutes prior to dosing with barium chloride to accelerate intestinal transit time) and a charcoal meal. Controls or animals given test

compound 15 minutes prior to testing, are then dosed with BaCl (300 mg/Kg per os) to accelerate intestinal transit time, and are then immediately administered charcoal meal. After 10 minutes, the mice are killed and the distance travelled by the charcoal meal measured.

Compounds of formula 1 show an inhibitory effect counteracting BaCl treatment, indicating activity against irritable bowel syndrome as illustrated in the below table.

| Dose (mg/kg p.o.) | % Augm. Transit after stimulation | % Inhibition induced by Compound* |
|---|---|---|
| 10 | + 127.6 | - 15.7 (NS) |
| 30 | + 127.6 | - 59.9 (p 0.05) |
| 30 | transit normal | - 33.7 (p 0.05) |

* is (S,RS)-2,6-dimethyl-3-carbomethoxy-4-(3-nitrophenyl)-5-[3-(4-[-(tetrahydropyran-2-yloxy)ethyl]phenoxy)propoxycarbonyl]-1,4-dihydropyridine.

## EXAMPLE 5

(Toxicology)

Oral dosing of dogs with (S,RS)-2,6-dimethyl-3-carbomethoxy-4-(3-nitro phenyl)-5-[3-(4-[-(tetrahydropyran-2-yloxy)ethyl]-phenoxy)propoxycarbonyl]-1,4 dihydropyridine for three months at 1.5 and 10 mg/kg/day did not produce any pathological change. Ophthalmoscopy remained normal throughout the study at all doses, as well as urine analysis.

**Claims**

1. Use of a compound of formula 1

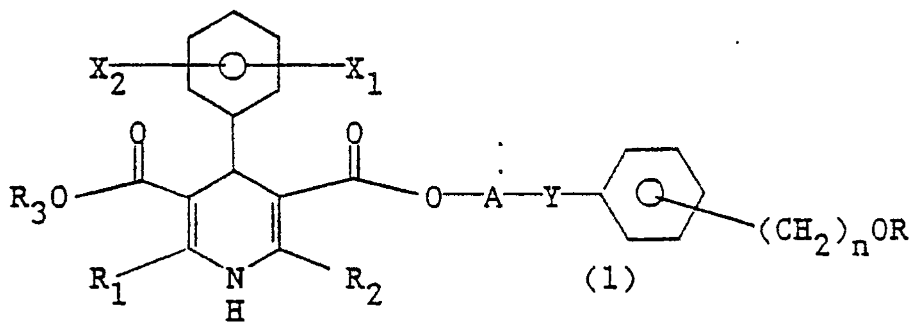

(1)

wherein
n is an integer from 1 to 4;
$R_1$ and $R_2$ are lower alkyl;
$R_3$ is lower alkyl or alkoxyalkyl;
A is alkylene of two to eight carbon atoms;
$X_1$ and $X_2$ are each independently $-NO_2$, $-CF_3$, $CH_3O-$, -CN, -H, lower alkyl or halo;
Y is -O-, -S-, -S(O)-, or $-S(O)_2-$; and
R is H, lower alkyl, cycloalkyl, alkoxyalkyl, cycloalkyloxy-alkyl, alkoxycycloalkyl, acyl, or saturated or unsaturated 5- or 6-membered heterocyclyl optionally substituted with lower alkyl or alkoxy, wherein the

heteroatom is one oxygen atom,

in the manufacture of a medicament suitable for treating irritable bowel syndrome or convulsive disorders, or protecting neural tissue from ischemic damage.

2. The use of claim 1, wherein said compound of formula 1 is (S,RS)-2,6-dimethyl-3-carbomethoxy-4-(3-nitrophenyl)-5-[3-(4-[2-(tetrahydropyran-2-yloxy)ethyl]phenoxy)propoxycarbonyl]-1,4-dihydropyridine.

3. An agent for treating irritable bowel syndrome, convulsive disorders, or protecting neural tissue from ischemic damage, which comprises a compound of formula 1 as defined in claim 1 or 2.

4. A composition adapted for treating irritable bowel syndrome, convulsive disorders, or protecting neural tissue from ischemic damage, which comprises a therapeutically effective amount of a compound of formula 1 as defined in claim 1 or 2, and a pharmaceutically acceptable excipient.

5. An irritable bowel syndrome, convulsive disorder, or neural tissue protection from ischemic damage agent for mammals, wherein a compound of formula 1 as defined in claim 1 or 2 is used as an active ingredient.

6. A process for the production of medicaments based on a compound of formula 1 as defined in claim 1 or 2 against irritable bowel syndrome, convulsive disorders, or protecting neural tissue from ischemic damage, wherein said compound of formula 1 is converted into an agent against irritable bowel syndrome, convulsive disorders, or protecting neural tissue from ischemic damage.

7. A process for preparing a composition for treating irritable bowel syndrome, convulsive disorders, or protection of neural tissue from ischemic damage for use in mammals, which comprises mixing a therapeutically effective amount of a compound of formula 1 as defined in claim 1 or 2 with a pharmaceutically acceptable excipient.